# EUROPÄISCHE PATENTSCHRIFT

(11) **EP 0 433 851 B1**
(45) Veröffentlichungstag und Bekanntmachung des Hinweises auf die Patenterteilung: **08.09.1993**
(21) Anmeldenummer: 90123826.1
(22) Anmeldetag: 11.12.1990
(51) Int. Cl.: A61F 2/02, A61F 2/00

(54) **Vorrichtung zum Befestigen von künstlichen Körperteilen, insbesondere von Gesichtsprothesen**
Device for fixing artificial members, especially a facial prosthesis
Dispositif pour fixer des membres artificiels, en particulier une prothèse faciale

(30) Priorität: 22.12.1989 DE 3942674
(43) Veröffentlichungstag der Anmeldung: 26.06.1991
(73) Patentinhaber: Oswald Leibinger GmbH, 78570 Mühlheim (DE)
(72) Erfinder: Leibinger, Karl, W-7200 Tuttlingen-Möhringen (DE); Leibinger, Franz, W-7202 Mühlheim-Stetten (DE)
(74) Vertreter: von Hellfeld, Axel, Dr. Dipl.-Phys.

(56) Entgegenhaltungen:
- DE-B- 1 075 791
- GB-A- 2 176 709
- LU-A- 59 918
- US-A- 4 805 602

## Beschreibung

Die Erfindung betrifft eine Vorrichtung zum Befestigen von künstlichen Körperteilen, insbesondere von Gesichtsprothesen.

Zur Befestigung von Epithesen (künstlichen Ohren, Nasen, Augen etc.) im Bereich des Gesichtes werden gemäß dem Stand der Technik in der Regel Hilfsmittel wie eine Brille oder eine Prothese verwendet.

Eine andere Lösung (DE-A-19 61 531 und DE-A-24 13 883) zum Befestigen von Epithesen im Bereich des Gesichtes sieht vor, daß einzelne Implantate im Schädelknochen fixiert werden, an denen dann die Epithese befestigt wird, wie bei Implantaten in der Mundhöhle zur Fixation von Zahnprothesen. Ein Nachteil eines solchen Implantatsystems liegt darin, daß genügend Knochen vorhanden sein muß, um die Implantatpfosten aufnehmen zu können. So ist es häufig nicht möglich, am Ort der zu befestigenden Epithese die Implantatpfosten zu setzen. Auch haben solche Implantatpfosten den Nachteil, daß sie ein sogenanntes "zweistufiges" Verfahren erfordern, wobei zunächst der Implantatpfosten in den Knochen eingefügt wird und dort eine längere Zeit ohne Belastung durch die Epithese verbleibt, um einheilen zu können. Erst danach wird mit zeitlichem Abstand von einigen Monaten dann auf dem oder den Implantatpfosten die Epithese bei einer zweiten Operation befestigt.

Auch haben solche Implantat-Systeme zum Befestigen von Epithesen den Nachteil, daß die Stellung und die Form der Epithese nicht immer optimal sein kann, da man beim Setzen der Implantate ungünstigen Randbedingungen unterliegt, d.h. die Implantate nicht immer dort setzen kann, wo sie für einen idealen Sitz der Epithese wünschenswert wären.

Der Erfindung liegt die Aufgabe zugrunde, eine Vorrichtung zum Befestigen von künstlichen Körperteilen, insbesondere von Gesichtsprothesen, an einem Träger zu schaffen, die dem Chirurgen eine weitgehende Gestaltungsfreiheit gibt, einen raschen Heilvorgang ermöglicht und einen sicheren und ästhetisch ansprechenden Sitz des künstlichen Körperteiles gewährleistet.

Erfindungsgemäß wird diese Aufgabe durch die Merkmale des Kennzeichnenden Teils des Anspruchs 1 gelöst.

An dem Draht bzw. Steg wird dann das künstliche Körperteil (die Epithese) mittels einer als solches bekannten Klemmvorrichtung befestigt.

Bevorzugt ist vorgesehen, daß auf dem genannten Drehteil eine Abdeckkappe befestigbar ist, mit welcher der Draht am Drehteil festklemmbar ist, so daß bei Verwendung mehrerer Pfosten und zugehöriger Drehteile die gesamte Anordnung einschließlich der Drehteile starr ist. Vor dem Festziehen der Abdeckkappe sind der Draht bzw. der Steg und das Drehteil noch beweglich, so daß sie der Operateur vor Ort an die gewünschte Geometrie anpassen kann.

In einer bevorzugten Ausgestaltung der Erfindung weist der Pfosten eine umlaufenden Nut zur Aufnahme von Gewebe auf, so daß die Vorrichtung insgesamt gut einwachsen kann.

Bevorzugt weist der Pfosten an seinem dem Träger (Platte oder insbesondere Gitter) einen Gewindestift auf, der in ein entsprechendes Gewinde im Träger einschraubbar ist.

Nachfolgend werden Ausführungsbeispiele der Erfindung anhand der Zeichnung näher beschrieben. Es zeigt:
- Fig. 1: einen Schnitt durch eine Vorrichtung zum Befestigen von Epithesen einschließlich eines Knochenstückes und eines Trägers;
- Fig. 2: ein Ausführungsbeispiel einer Vorrichtung zum Befestigen der Epithese an einem Träger in auseinandergezogenem Zustand;
- Fig. 3: ein anderes Ausführungsbeispiel einer Vorrichtung zum Befestigen einer Epithese an einem Träger;
- Fig. 4: ein weiteres Ausführungsbeispiel einer Vorrichtung zum Befestigen einer Epithese an einem Träger und
- Fig. 5: einen Träger in Draufsicht.

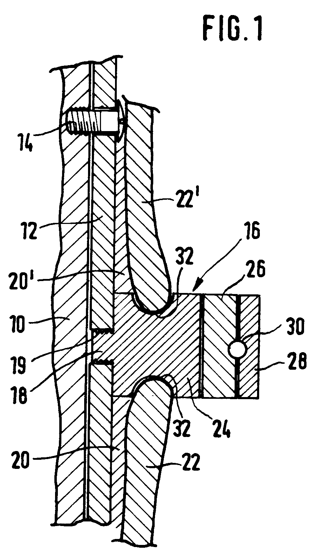

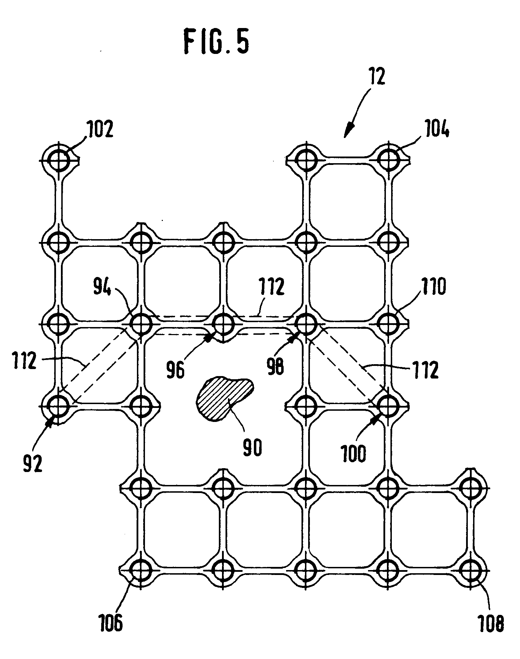

Fig. 1 zeigt im Schnitt ein Knochenstück 10, beispielsweise benachbart einem Gehörgang, über dem ein künstliches Ohr befestigt werden soll. Auf dem Knochen 10 ist ein gitterförmiger Träger 12 befestigt, der in Fig. 5 näher dargestellt ist. Der gitterförmige Träger 12 weist eine Vielzahl von Löchern auf. Durch eines der Löcher ist eine Schraube 14 geführt, die in den Knochen 10 eingeschraubt ist. In Fig. 1 ist schematisch nur eine einzige Schraube 14 gezeigt. Darüberhinaus ist der Träger 12 mit mehreren Schrauben im Knochen 10 verankert, wie weiter unten anhand des Ausführungsbeispieles gemäß Fig. 5 näher erläutert ist.

Auf dem fest im Knochen 10 verankerten Träger 12 ist eine Befestigungsvorrichtung 16 befestigt, die Gegenstand der Erfindung ist. Die Befestigungsvorrichtung 16 weist einen Gewindestift 18 auf, der in ein entsprechendes Gewinde in einem Loch 19 im Träger 12 eingeschraubt ist.

Im verheilten Zustand wächst Knochen in Lücken des Trägers (vergleiche Fig. 5) und kann diesen teilweise überdecken. Solch gewachsener Knochen ist in Fig. 1 mit dem Bezugszeichen 20 angedeutet.

Gewebe 22 liegt über dem Träger 12 und ggf. dem Knochen 20 und umschließt nach Ablauf des Einwachsens und des Heilprozesses die Befestigungsvorrichtung 16.

Die Befestigungsvorrichtung 16 weist einen Pfosten 24 auf, in dem eine umlaufende Nut 32 ausgeformt ist, in welche das Gewebe 22, 22' dicht einwächst. Der Pfosten 24 ist mittels des Gewindestiftes 18 fest in den Träger 12 eingeschraubt, kann sich also in bezug auf den Träger nicht bewegen, insbesondere nicht drehen.

Auf dem Pfosten 24 ist ein Drehteil 26 so befestigt, daß es zunächst in bezug auf den Pfosten 24 um die gemeinsame Längsachse der Teile drehbar ist. Über dem Drehteil 26 ist eine Abdeckkappe 28 montiert. Im Drehteil 26 und in der Abdeckkappe 28 ist eine durchgehende Aufnahme 30 ausgeformt zur Aufnahme eines Drahtes. Die Funktion des Drahtes bzw. Steges ist weiter unten anhand der Fig. 5 näher erläutert.

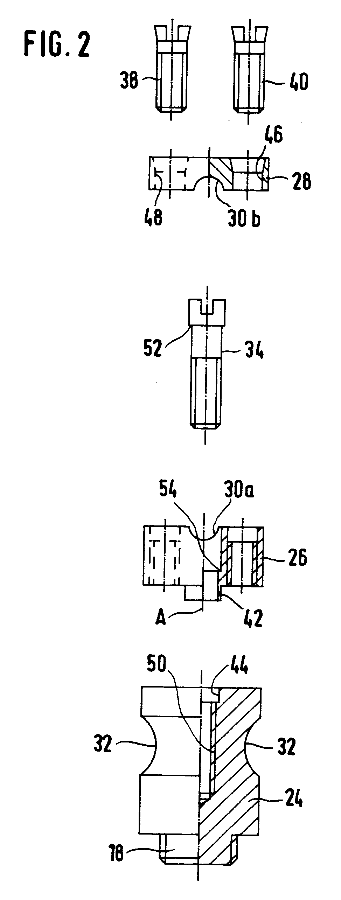

Die Fig. 2, 3 und 4 zeigen verschiedene Ausführungsbeispiele für eine Befestigungsvorrichtung 16.

Beim Ausführungsbeispiel gemäß Fig. 2 wird das Drehteil 26 auf dem Pfosten 24 mittels einer Schraube 34 so befestigt, daß es um die Längsachse A der Teile drehbar ist. Mittels Schrauben 38, 40 wird die Abdeckkappe 28 auf dem Drehteil 26 befestigt. Im zusammengebauten Zustand ist die Schraube 34 im Drehteil 26 so weit versenkt, daß die Aufnahme 30a frei bleibt, d.h. der Kopf der Schraube 34 ragt nicht in die Aufnahme 30a.

An seinem unteren Ende weist das Drehteil 26 einen Vorsprung 42 auf, der in eine komplementäre Ausnehmung 44 im Pfosten 24 paßt, so daß das Drehteil 26 frei in bezug auf den Pfosten 24 drehen kann.

Die Schrauben 38, 40 durchsetzen Bohrungen 46 bzw. 48 in der Abdeckkappe 28.

Die Länge einer Bohrung 50 im Pfosten 24, die Höhe des Drehteiles 26 und die Länge der Schraube 34 sind so bemessen, daß in zusammengebautem Zustand ein Anschlag 52 am Kopf der Schraube 34 direkt oberhalb eines entsprechenden Anschlages 54 im Drehteil 26 positioniert ist, derart, daß das Drehteil 26 zwar eine Drehbewegung, aber keine Bewegung in Richtung der Längsachse A ausführen kann.

In der Abdeckkappe 28 ist eine der Aufnahme 30a im Drehteil 26 entsprechende, ebenfalls halbkreisförmige Aufnahme 30b ausgeformt, so daß in zusammengebautem Zustand insgesamt eine im Schnitt etwa kreisförmige Aufnahme gemäß Fig. 1 entsteht, in welcher ein Draht bzw. Steg zunächst locker positionierbar und nach Festziehen der Schrauben 38, 40 starr mit dem Drehteil 26 verbindbar ist.

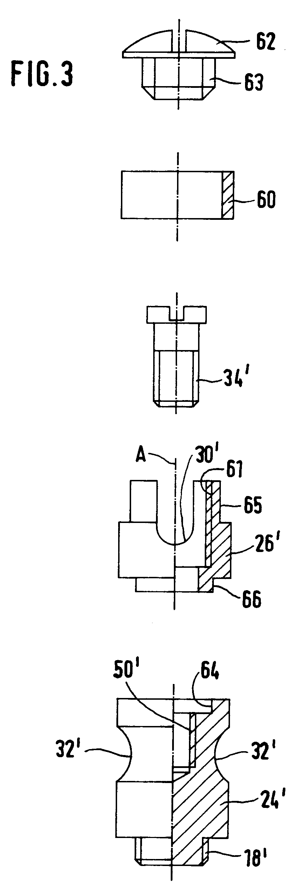

Fig. 3 zeigt ein weiteres Ausführungsbeispiel einer Befestigungsvorrichtung 16.

Beim Ausführungsbeispiel gemäß Fig. 3 sind dem Ausführungsbeispiel gemäß Fig. 2 entsprechende Bauteile mit gleichen Bezugszahlen versehen, die jeweils zusätzlich einen Strich aufweisen. Das Drehteil 26' ragt mit einem Vorsprung 66 in eine komplementäre Ausnehmung 64 im Pfosten 24'. Eine Schraube 34' durchgreift das Drehteil 26' und ist in einer Bohrung 50' im Pfosten 24' verschraubt. Dabei bleibt das Drehteil 26' noch für eine Drehung um die Achse A frei.

Eine Hülse 60 paßt über einen verjüngten Abschnitt 65 des Drehteiles 26'. Eine Schraube 62 durchgreift die Hülse 60 und ist mit einem Außengewinde 63 in einem Innengewinde 61 des verjüngten Abschnittes 65 verschraubbar. Beim Anziehen der Schraube 26 in bezug auf das Drehteil 26' kann ein in der Aufnahme 30' angeordneter Draht mittels der Hülse 60 verklemmt werden.

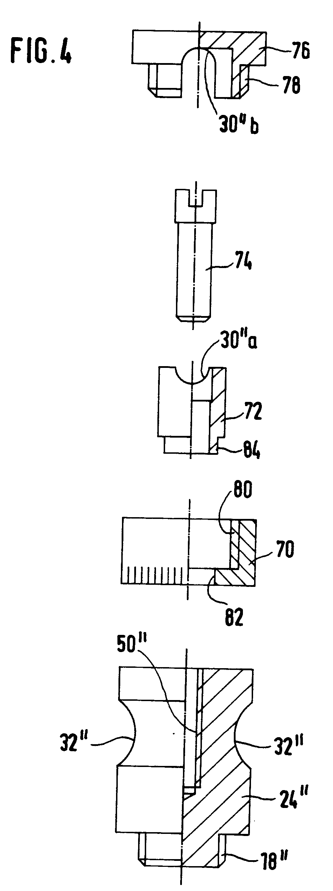

Fig. 4 zeigt eine Abwandlung einer Befestigungsvorrichtung 16 (Fig. 1). Beim Ausführungsbeispiel gemäß Fig. 4 sind dem Ausführungsbeispiel gemäß Fig. 2 entsprechende Bauteile mit gleichen Bezugszahlen versehen, die jeweils zwei Striche aufweisen.

Über einem Pfosten 24'' ist eine Hülse 70 befestigt, und zwar mittels einer Schraube 74, die ein Drehteil 72 durchgreift und in eine entsprechende Bohrung 50'' im Pfosten 24'' eingeschraubt ist. Das Drehteil 72 greift mit einem Vorsprung 84 in eine entsprechende Ausnehmung 82 in der Hülse 70. In zusammengebautem Zustand liegt die Aufnahme 30''a frei, d.h. der Kopf der Schraube 74 ist in dem Drehteil 72 versenkt. Eine Kappe 76 ist mit einem Außengewinde 78 versehen, welches mit einem Innengewinde 80 in der Hülse 70 zusammenwirkt. Hierzu ist die Hülse 70 außen mit einer Riffelung versehen, so daß durch Drehung der Hülse 70 in bezug auf die Kappe 76 letztere festgezogen wird. Dabei kann ein in die Ausnehmungen 30''a im Drehteil 72 bzw. 30''b in der Kappe 76 eingelegter Draht bzw. Steg festgezogen werden.

Fig. 5 zeigt eine Draufsicht auf einen Träger 12, der in Fig. 1 nur ausschnittweise dargestellt ist.

Der Träger 12 ist gitterförmig ausgebildet und an den Positionen 92, 94, 96, 98 und 100 sind jeweils Befestigungsvorrichtungen 16 gemäß den Fig. 1 bis 4 montiert.

Der gitterförmige Träger 12 ist an den Positionen 102, 104, 106, 108 und 110 jeweils mit einer Schraube 14 (vergleiche Fig. 1) am Knochen 10 befestigt. Mittels des in Fig. 5 gezeigten Trägers 12 soll beispielsweise ein künstliches Ohr über einem Gehörgang 90 befestigt werden. Hierzu wird ein Draht 112 durch die Aufnahmen 30 in den einzelnen Befestigungsvorrichtungen 16 (an den genannten Positionen 92, 94, 96, 98 und 100) befestigt. Aufgrund der oben beschriebenen Drehbarkeit der Drehteile kann der Draht zunächst vom Chirurgen eingelegt werden und dann so wie gewünscht gebogen werden. Danach kann die oben beschriebene Fixierung des Drahtes in bezug auf die Drehteile erfolgen, so daß insgesamt eine dreidimensional stabile Anordnung entsteht. Auf dem Draht 112 kann dann das künstliche Ohr in bekannter Weise mit Klemmen befestigt werden.

## Patentansprüche

1. Vorrichtung zum Befestigen von künstlichen Körperteilen, insbesondere einer Gesichtsprothese, an einem Träger (12), der seinerseits an einem Knochen (10) befestigbar ist, mit einem starr mit dem Träger (12) verbindbaren Pfosten (24),
dadurch **gekennzeichnet,** daß der Träger (12) als Platte oder Gitter ausgebildet ist, daß auf dem Pfosten (24) ein Drehteil (26, 72) drehbar befestigt ist, das eine Aufnahme (30) für einen Draht (112) aufweist, und daß der Draht (112) am Drehteil (26, 72) festklemmbar ist.

2. Vorrichtung nach Anspruch 1,
dadurch **gekennzeichnet,** daß die Festklemmung des Drahtes (112) am Drehteil (26, 72) mittels einer auf dem Drehteil befestigten Abdeckkappe (28) erfolgt.

3. Vorrichtung nach einem der Ansprüche 1 oder 2,
dadurch **gekennzeichnet,** daß der Pfosten (24) eine umlaufenden Nut (32) zur Aufnahme von Gewebe aufweist.

4. Vorrichtung nach einem der vorhergehenden Ansprüche,
dadurch **gekennzeichnet,** daß der Pfosten (24) einen Gewindestift (18) aufweist.

5. Vorrichtung nach einem der vorhergehenden Ansprüche,
dadurch **gekennzeichnet,** daß der Träger (12) ein Gitter ist, in welches der Pfosten mit dem Gewindestift (18) einschraubbar ist.

## Claims

1. A device for fastening artificial body parts, especially a facial prosthesis to a support member (12) which in turn is adapted to be fastened to a bone (10), comprising a post (24) adapted for rigid connection to the support member (12), **characterized** in that the support member (12) is embodied by a plate or lattice, that a rotary member (26, 72) is rotatably mounted on the post (24) and includes a mounting facility (30) for a wire (112), and that the wire (112) is adapted to be clamped to the rotary member (26, 72).

2. The device as claimed in claim 1, characterized in that the clamping of the wire (112) at the rotary member (26, 72) is effected by a cover cap (28) fastened on the rotary member.

3. The device as claimed in claim 1 or 2, characterized in that the post (24) is formed with a circumferential groove (32) to receive tissue.

4. The device as claimed in any one of the preceding claims, characterized in that the post (24) comprises a threaded pin (18).

5. The device as claimed in any one of the preceding claims, characterized in that the support member (12) is a lattice into which the post is adapted to be screwed by its threaded pin (18).

## Revendications

1. Dispositif pour fixer des organes artificiels du corps, en particulier une prothèse faciale, sur un support (12) qui, de son côté, peut se fixer à un os (10) dispositif présentant un pilier (24) qui peut se relier rigidement avec le support (12),
dispositif caractérisé par le fait que le support (12) est congru sous forme de plaquette ou de grille, par le fait que sur le pilier (24) est fixée, avec liberté de rotation, une pièce rotative (26, 72) qui présente un réceptacle (30) pour un fil métallique (112), et par le fait que le fil métallique (112) peut se bloquer sur la pièce rotative (26, 72 ).

2. Dispositif selon la revendication 1,
caractérisé par le fait que le blocage du fil métallique (112) sur la pièce rotative (26, 72) se fait au moyen d'un capuchon de recouvrement (28) fixé sur la pièce rotative.

3. Dispositif selon l'une des revendications 1 ou 2,
caractérisé par le fait que le pilier (24) présente une rainure périphérique (32) pour recevoir du tissu.

4. Dispositif selon l'une des revendications précédentes,
caractérisé par le fait que le pilier (24) présente un embout fileté (18).

5. Dispositif selon l'une des revendications précédentes,
caractérisé par le fait que le support (12) est une grille dans laquelle le pilier peut se visser par l'embout fileté (18).
